(19) Europäisches Patentamt
European Patent Office
Office européen des brevets

(11) Numéro de publication: **0 092 463**
**B1**

(12) # FASCICULE DE BREVET EUROPEEN

(45) Date de publication du fascicule du brevet: **25.09.85**

(51) Int. Cl.⁴: **C 07 C 43/13**, C 07 C 41/28

(21) Numéro de dépôt: **83400721.3**

(22) Date de dépôt: **12.04.83**

(54) **Procédé de fabrication de monoéther de monoéthylèneglycol par hydrogénolyse d'acétals cycliques.**

(30) Priorité: **20.04.82 FR 8206728**

(43) Date de publication de la demande:
**26.10.83 Bulletin 83/43**

(45) Mention de la délivrance du brevet:
**25.09.85 Bulletin 85/39**

(84) Etats contractants désignés:
**AT BE CH DE FR GB IT LI LU NL SE**

(56) Documents cités:
**BE - A - 675 033**
**FR - A - 1 409 152**
**FR - A - 2 422 614**

**JOURNAL OF THE CHEMICAL SOCIETY, CHEMICAL COMMUNICATIONS, no. 3, février 1981, pages 106-108, M. BARTOK et al.: "Isomerization and hydrogenolysis of 1,3-dioacycloalkanes on metals catalysts"**

(73) Titulaire: **ATOCHEM, 12/16, allée des Vosges, F-92400 Courbevoie (FR)**

(72) Inventeur: **Ghenassia, Elie François Marius, 30, route de Lapugnoy, F-62920 Chocques (FR)**
Inventeur: **Lakodey, André Jean, 37, rue de Lillers, F-62920 Chocques (FR)**

**EP 0 092 463 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé sélectif de fabrication des monoéthers de glycol et plus particulièrement les monoéthers de monoéthylèneglycol.

La fabrication de ces produits s'effectue de façon connue par réaction entre un alcool et l'oxyde d'éthylène, en phase liquide, en présence d'un catalyseur. Dans ce type de procédé, le monoéther du monoéthylèneglycol formé présentant vis-à-vis de l'oxyde d'éthylène la même réactivité que l'alcool de départ réagit dès sa formation, compétitivement à celui-ci, avec l'oxyde d'éthylène pour donner le monoéther du diéthylèneglycol qui, à son tour, conduit à la formation de monoéther des oligoéthylèneglycols suivant une distribution statistique. En outre, la proportion de ces dérivés d'oligoéthylèneglycol augmente considérablement avec le poids moléculaire de l'alcool mis en œuvre, si bien qu'il est industriellement impossible d'assurer une fabrication des monoéthers de monoéthylèneglycol dérivés d'alcools comportant plus de deux atomes de carbone sans une formation importante de sous-produits.

L'hydrogénolyse des acétals ouverts conduit à la formation simultanée et équimoléculaire d'éther et d'alcool suivant la réaction:

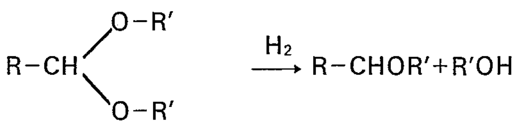


Cette réaction est connue depuis les travaux de Cabanac – C.R. de l'Académie des Sciences Française, 1929, 1er semestre, T. 188, N° 19, page 1251. Le brevet français 2 422 614 décrit un procédé amélioré d'hydrogénolyse d'acétals et de cétals d'hydrocarbures organiques, en opérant en présence d'un catalyseur constitué d'un halogénure d'un élément du groupe III A tels que $AlCl_3$, $BF_3$, etc., et d'un catalyseur d'hydrogénation constitué uniquement de platine ou de rhodium déposé sur un support, en phase liquide, à une température comprise entre −15° et +125° C, sous une pression comprise entre 3,5 et 140 atomosphères.

Selon l'invention, il est possible de fabriquer les alcoxy-2 éthanol, de formule générale

$$R-CH_2-O-CH_2-CH_2OH$$

de façon sélective par hydrogénolyse des acétals dérivés de l'éthylèneglycol et d'aldéhydes aliphatiques linéaires ou ramifiés ou alkylcycloaliphatiques, correspondant au groupement R, et comportant entre 1 et 12 atomes de carbone.

Ces acétals présentent la structure cyclique d'un dioxolanne 1-3:

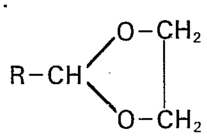

dans lequel R est un groupement hydrocarboné saturé aliphatique, linéaire ou ramifié, cycloaliphatique ou aromatique, substitué ou non par d'autres groupements hydrocarbonés, le nombre de carbone de la chaîne principale étant compris entre 1 et 12 et celui de chacun des substituants entre 1 et 4. En pratique, R est l'hydrogène ou un radical méthyl, éthyl, propyl, isopropyl, butyl, isobutyl, tertiobutyl, pentyl, hexyl, heptyl, octyl, etc., ou un de leurs isomères, substitués ou non. R peut être aussi un radical alicyclique tels cyclohexyl, cyclohexenyl, etc., substitué ou non, ou un groupement aromatique benzyl, tolyl, etc.

Les acétals qui permettent d'obtenir simultanément les meilleurs taux de conversion et des sélectivités élevées sont les alkyldioxolannes provenant de la réaction entre un aldéhyde R – CHO et un diol vicinal, plus particulièrement l'éthylèneglycol.

L'hydrogénolyse de dioxolanne de l'invention s'effectue en phase liquide en présence d'un système co-catalytique constitué par un catalyseur d'hydrogénation et un catalyseur acide, le premier fonctionnant en tant que catalyseur hétérogène et le second étant soit adsorbé sur le support du précédent ou sur tout autre support, soit dissous dans le mélange à hydrogénolyser, soit un équilibre entre les deux phases et procède selon les principes bien connus de la catalyse homogène acide.

Les catalyseurs d'hydrogénation sont de façon générale les métaux de transition du groupe VIII de la classification périodique. Il a cependant été constaté que dans le cas de la présente invention, l'hydrogénolyse doit être effectuée en présence de palladium, les autres métaux du groupe VIII ne permettant pas d'obtenir de résultat industriellement intéressant. Le palladium peut être introduit dans le milieu réactionnel tel quel en poudre finement divisée, ou déposé sur un support comme l'alumine, le charbon, la silice, l'oxyde de titane, le Kieselguhr et autres.

Le catalyseur acide associé au palladium est choisi parmi les acides du phosphore tels que les acides phosphorique, pyrophosphorique, métaphosphorique, phosphonique, alkylphosphonique, ou encore les esters phosphoriques, y compris pyrophosphoriques, de l'éthylèneglycol. Bien que d'autres acides forts aient été essayés comme cocatalyseur du palladium, il n'a pas été possible d'obtenir de résultat industriellement intéressant.

Avec le système co-catalytique palladium-acide du phosphore, il est possible d'obtenir, dans les meilleures conditions opératoires, d'excellents taux de conversion des dioxolannes-1,3 pouvant être souvent supérieurs à 99%, et des sélectivités élevées, souvent supérieures à 95%, en monoéther du monoéthylèneglycol.

La réaction d'hydrogénolyse des dioxolannes-1,3 est conduite en phase liquide en présence d'éthylèneglycol comme solvant. Il a été constaté que l'utilisation de solvant autre que l'éthylèneglycol entraîne la formation de sous-produits qui limitent de façon sensible la sélectivité de la réaction, réduisent le rendement en monoéther de monoéthylèneglycol et qui peuvent rendre plus difficile la séparation par distillation des éthers de glycol souhaités. Les quantités d'éthylèneglycol qu'il est souhaitable de mettre en œuvre varient en fonction

de la masse moléculaire du dioxolanne à hydrogénolyser. Le rapport molaire monoéthylèneglycol sur dioxolanne 1-3 peut varier entre 1 et 15 et doit de préférence se situer entre 1 et 10 ce qui permet de concilier à la fois une sélectivité élevée et une productivité industrielle intéressante en monoéther de glycol.

La quantité de palladium nécessaire à l'hydrogénolyse n'est pas critique, toutefois bien qu'il n'apparaisse pas d'effet nuisible, il n'est pas avantageux d'utiliser un trop fort excès de catalyseur. Il est recommandé d'avoir une concentration en palladium, par rapport à la masse totale du mélange dioxolanne 1-3-éthylèneglycol comprise entre 50 et 1500 ppm et de préference entre 100 et 1000 ppm.

La teneur en acide du phosphore ou en ester phosphorique du monoéthylèneglycol rapporté en $H_3PO_4$ peut varier entre 0,01 et 0,75% et préférentiellement entre 0,02 et 0,5% de la masse du mélange acétal – éthylèneglycol.

Il apparaît que l'emploi d'un stabilisant de la famille de l'hydroquinone à raison de 0,0005-0,005% en poids et en particulier de 0,001 à 0,003% par rapport au poids du mélange dioxolanne–solvant exerce une influence bénéfique sur la sélectivité en monoéther du monoéthylèneglycol; la quantité de sous-produits autres que le dibutoxy 1-2 éthane, par exemple, formés pendant l'hydrogénolyse du propyl-2 dioxolanne 1-3 pouvant être divisée par un facteur 2 à 3.

Les températures d'hydrogénolyse les plus favorables varient entre 100 et 250° C. On préfère cependant opérer entre 150 et 200° C de façon à obtenir les meilleurs taux de conversion des dioxolannes 1-3 en des temps courts et sans mettre en œuvre des quantités trop importantes de catalyseur. La pression d'hydrogène dans le réacteur peut varier entre 20 et 150 bar bien que des pressions de 30 à 100 bar soient préférables. Dans ces conditions, la durée de l'hydrogénolyse n'excède pas, en opérant en discontinu, des durées de l'ordre de 3 à 5 h.

La préparation des dioxolannes peut s'effectuer suivant l'une des nombreuses méthodes connues. La plus simple consiste à cétaliser un aldéhyde R–CHO par l'éthylèneglycol avec élimination de l'eau formée dans la réaction par les moyens classiques de la distillation fractionnée ou azéotropique ou à l'aide d'un agent desséchant. Les mélanges d'aldéhydes de structure R–CHO, R étant un des radicaux définis précédemment, et d'éthylèneglycol, peuvent être hydrogénolysés *in situ* après une période d'agitation de 2 à 3 h à la température de 80 à 100° C en atmosphère inerte. L'hydrogénolyse est conduite selon les conditions de température et de pression précédemment citées, après introduction d'hydrogène, le système co-catalytique et éventuellement l'hydroquinone ayant été introduits initialement dans le réacteur avec le mélange aldéhyde – éthylèneglycol. Les produits réactionnels sont principalement constitués de monoéther de glycol et de dioxolanne dérivés de l'aldéhyde. L'alcool susceptible de se former par hydrogénation directe de l'aldéhyde et malgré la présence d'eau n'est retrouvé qu'en faible quantité.

L'éther de glycol contenu dans le mélange réactionnel après hydrogénolyse peut être séparé des autres constituants par tous les moyens classiques connus, tels que par exemple la distillation fractionnée après élimination du catalyseur hétérogène par filtration.

Les exemples suivants, sans être limitatifs, illustrent l'invention.

*Exemple 1*

Dans un autoclave en acier inoxydable NSMC d'une capacité de 1 l agité mécaniquement, on introduit 250 g d'éthylèneglycol et 50 g (0,431 mol) de propyl-2 dioxolanne 1-3

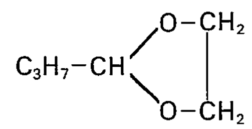

1 g de catalyseur constitué de 5% en poids de palladium déposé sur alumine, 180 mg d'acide phosphorique concentré et 3 mg d'hydroquinone. Après avoir purgé l'autoclave avec de l'azote, ce dernier est pressurisé par de l'hydrogène de façon que la pression intérieure atteigne 100 bar à la température de 150° C. La pression est ensuite ajustée à 100 bar avec de l'hydrogène selon les besoins en maintenant la température à 150° C pendant 3 h. Après refroidissement, l'autoclave est vidé et le mélange réactionnel filtré sous atmosphère d'azote. L'analyse par chromatographie en phase gazeuse C.P.G. du filtrat montre que le taux de conversion total du propyl-2 dioxolanne 1-3 atteint 99,2%. Le poids de butoxy-2 éthanol-1 formé est de 48,5 g (0,411 mol) et celui de dibutoxy-1-2 éthane de 1,36 g (0,0078 mol).

*Exemple 2 – Comparatif*

On procède selon l'exemple 1 avec 1 g de catalyseur constitué de 5% en poids de rhodium sur support alumine en présence de 180 mg d'acide phosphorique concentré. L'hydrogénolyse de 50 g de propyl-2 dioxolanne 1-3 en présence de 250 g d'éthylèneglycol contenant 3 mg d'hydroquinone donne, après 3 h d'agitation sous une pression de 100 bar à la température de 200° C et filtration, un taux de conversion du propyldioxolanne (PDX) de 57% seulement. Le mélange réactionnel contient 14,7 g de butoxy-2 éthanol-1 et 5,6 g de butanol normal.

*Exemples 3 et 4 – Comparatifs*

Les essais correspondant à ces exemples sont effectués dans les conditions de l'exemple 1 en utilisant pour chacun d'eux 1 g de catalyseur constitué par du platine déposé sur alumine à raison de 5% en poids, ou du nickel déposé sur Kieselguhr à raison de 50% de nickel en poids.

Le tableau suivant regroupe les quantités des principaux produits obtenus.

| | Exemple N° 3 | Exemple N° 4 |
|---|---|---|
| Catalyseur d'hydrogénation | $Pt/Al_2O_3$ | Ni/Kieselguhr |
| Co-catalyseur | $H_3PO_4$ | $H_3PO_4$ |
| Taux de conversion du PDX (%) | 38 | 51,6 |
| Poids de butoxy-2 éthanol (g) | 4,98 | 11,3 |
| Poids de butanol (g) | 5 | 6,4 |

*Exemple 5*

On opère selon les conditions de l'exemple 1: 50 g (0,431 mol) de propyl-2 dioxolanne 1-3 et 250 g d'éthylèneglycol sont hydrogénolysés en présence du couple catalytique constitué par 5 g de palladium déposé sur oxyde de titane à raison de 0,2% en poids et 180 ml d'acide phosphorique concentré. Le rendement en butoxy-2 éthanol-1 atteint 92%.

*Exemple 6*

Dans l'autoclave de 1 l utilisé dans l'exemple 1, 50 g de propyl-2 dioxolanne et 250 g d'éthylèneglycol sont introduits puis on ajoute 80 mg d'acide pyrophosphorique $H_4P_2O_7$, 1 g de catalyseur d'hydrogénation $Pd/Al_2O_3$ à 5% de métal actif en poids et 0,009 g d'hydroquinone. L'autoclave après purge à l'azote est pressurisé sous une pression de 100 bar d'hydrogène à la température de 170° C. Cette pression est maintenue 3 h sous agitation à 170° C. Après filtration, l'analyse chromatographique montre que 99,7% du propyldioxolanne sont transformés. Le taux de transformation de:

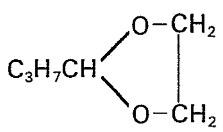

en butoxy-2 éthanol-1 (exprimé en mol) atteint 90,6% soit 46,1 g, les quantités de dibutoxy 1-2 éthane et de butanol normal sont respectivement de 2,21 et 1,35 g.

*Exemple 7 – Comparatif*

On opère selon les conditions de l'exemple 1. L'hydrogénolyse de 50 g de propyl-2 dioxolanne 1-3 en présence de 250 g de monoéthylèneglycol est effectuée en présence de 1 g de catalyseur au palladium déposé sur alumine à raison de 5% de métal actif en poids. Le co-catalyseur utilisé est l'acide paratoluène sulfonique à raison de 0,72 g pour les 300 g de mélange PDX – éthylèneglycol contenant 6 mg d'hydroquinone. Sous une pression d'hydrogène de 100 bar, l'autoclave est agité 3 h en maintenant la température à 150° C. La quantité de butoxy-2 éthanol-1 formée est de 37,5 g (0,318 mol), celles du dibutoxy-1-2 éthane et du butanol normal sont respectivement de 1,6 g (0,0091 mol) et de 0,5 g (0,0067 mol). L'utilisation de l'acide paratoluènesulfonique comme co-catalyseur entraîne la formation de quantités importantes de sous-produits lourds dont 6,7 g de diéthylèneglycol.

*Exemples 8 à 12 – Comparatifs*

Dans cette série d'exemples sont regroupés les essais effectués avec différents acides minéraux ou organiques. Les quantités de réactifs, propyldioxolanne, éthylèneglycol, hydroquinone et catalyseurs d'hydrogénation Pd sur charbon à raison de 5%, sont identiques à celles utilisées dans l'exemple 6. La durée de l'hydrogénolyse est de 3 h et la pression d'hydrogène de 100 bar. Les conditions particulières et les résultats sont rassemblés dans le tableau général suivant.

| N° exemple | Catalyseur acide | Poids acide (g) | T °C | Taux de conversion du PDX (%) | TT en butoxy-éthanol (%) | TT% en dibutoxy-éthane | TT% en butanol |
|---|---|---|---|---|---|---|---|
| 8 | HCl | 0,18 | 170 | 83,9 | 71 | 4,2 | 5,2 |
| 9 | HCOOH | 0,18 | 100 | 28,7 | 24,1 | 0,3 | 2,2 |
| 10 | Benzène phosphorique | 0,18 | 170 | 66 | 44 | 1,4 | — |
| 11 | $CF_3COOH$ | 0,18 | 170 | 65 | 60,3 | 3,2 | 0,2 |
| 12 | COOH<br>COOH | 0,18 | 170 | 11,5 | 8,8 | — | 1,3 |

TT% = Taux de transformation molaire du PDX en un des produits de réaction.

*Exemple 13*

Le mélange éthylèneglycol (250 g) – acide phosphorique concentré (180 mg) est chauffé pendant 3 h à 170° C. Le dosage chimique montre que l'acide introduit a été estérifié par l'éthylèneglycol. Ce mélange est introduit dans le réacteur

avec 50 g de propyldioxolanne et l'ensemble est hydrogénolysé en présence de 1 g de palladium déposé sur charbon et 0,009 g d'hydroquinone, à la température de 170° C sous 100 bar d'hydrogène, pendant 3 h. Après refroidissement et filtration, l'analyse montre que 96% de propyldioxolanne-1,3 sont transformés et que 90% l'ont été en butoxy-2 éthanol-1. La quantité de dibutoxyéthane atteint 1,74 g (0,01 mol).

*Exemple 14*

Le mélange de 50 g de

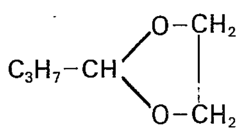

et 250 g de $CH_2OH-CH_2OH$ est hydrogénolysé en présence d'un système catalytique constitué par 1 g de palladium déposé sur alumine à raison de 5% en poids de Pd et 88 mg d'acide orthophosphorique concentré à 85%, et de 3 mg d'hydroquinone. Après 7 h d'agitation à la température de 185° C sous une pression d'hydrogène maintenue constante de 100 bar, 97,3% du propyldioxolanne sont transformés, 47,2 g (0,4 mol) de butoxy-2 éthanol-1 sont dosés ainsi que de très faibles quantités de butanol (0,008 mol) et de dibutoxy-1,2 éthane (0,003 mol).

*Exemple 15*

Un mélange de 50 g de

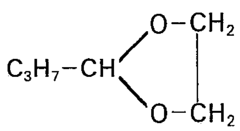

et 250 g d'éthylèneglycol est hydrogénolysé dans un autoclave en acier inoxydable en présence d'un système catalytique constitué par 1 g de palladium déposé sur alumine à raison de 5% en poids de métal actif, 1,76 g de $H_3PO_4$ à 85% et de 9 mg d'hydroquinone. Après 7 h d'agitation à la température de 100° C sous une pression maintenue constante de 100 bar, 75,4% du propyldioxolanne sont convertis. Le taux de transformation en butoxy-2 éthanol-1 atteint 70,6% et le rendement molaire 93,6%. Les quantités de butanol et de dibutoxy-1 éthane sont respectivement de 0,4 et 0,75 g.

*Exemple 16 — Comparatif*

Dans un réacteur de 1 l de capacité, agité mécaniquement, 300 g (2,58 mol) de propyl-2 dioxolanne 1-3 titrant 99,8% de pureté sont introduits avec 3 mg d'hydroquinone, 1 g de palladium sur alumine à raison de 5% en poids de Pd et 88 mg d'acide phosphorique à 85%. Après pressurisation par l'hydrogène, l'autoclave est chauffé à 185° C et la pression ajustée et maintenue à 100 bar pendant 3 h. Après refroidissement et filtration du catalyseur, le mélange réactionnel analysé par chromatographie en phase gazeuse contient 160 g (1,359 mol) de butoxy-2 éthanol-1, 90,9 g de dibutoxy-1-2 éthane (0,522 mol) et 33 g d'éthylèneglycol (0,53 mol). Le taux de transformation molaire du PDX en $C_4H_9-O-CH_2OH$ est de 52,7%.

*Exemple 17*

En opérant selon l'exemple 16, un mélange de 50 g de propyl-2 dioxolanne 1-3 et 250 g d'éthylèneglycol est hydrogénolysé dans un réacteur agité. Le couple catalytique est constitué de 1 g de $Pd/Al_2O_3$ contenant 5% en poids de métal actif et 200 mg d'acide orthophosphorique à 85%. Après addition de 3 ppm d'hydroquinone, l'autoclave est pressurisé par de l'hydrogène et la température contrôlée à 150° C. La pression est maintenue à 100 bar par appoint d'hydrogène, pendant 3 h. Après refroidissement et filtration le mélange réactionnel est dosé par CPG, 99,2% du propyldioxolanne est transformé et le TT de ce réactif en butoxy-2 éthanol-1 atteint 95,9%. La teneur en butanol dans le mélange réactionnel est de 0,31% et celle en butoxyéthane 0,12%.

*Exemple 18 — Comparatif*

On opère selon l'exemple 17 en remplaçant l'éthylèneglycol par du butanol normal, 50 g (0,431 mol) de propyldioxolanne et 250 g de butanol normal (3,78 mol) sont hydrogénolysés. Après 3 h d'agitation à 150° C sous 100 bar d'hydrogène, 98,9% de l'acétal sont transformés et le taux de transformation (TT%) en butoxy-1 éthanol-2 atteint 23,10% seulement et le TT en produit principal, le dibutyléther, s'élève à 71,6%.

*Exemple 19 — Comparatif*

On opère comme dans l'exemple 17 en remplaçant l'éthylèneglycol par du butoxy-2 éthanol-1. Après 3 h à 150° C sous 100 bar d'hydrogène, 99,9% de

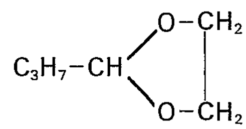

sont transformés et le taux de transformation de ce réactif en butoxy-2 éthanol-1 atteint seulement 32,7% et le TT en produit principal, le dibutoxy 1-2 éthane est de 62,2%.

*Exemple 20*

En opérant suivant l'exemple 17, le mélange soumis à l'hydrogénolyse contient 81,7 g (0,704 mol) de propyl-2 dioxolanne 1-3 et 218,3 g (3,521 mol) d'éthylèneglycol, ce qui correspond à un rapport molaire monoéthylèneglycol sur acétal de 5. Le couple catalytique est constitué par 1 g de palladium déposé sur charbon contenant 5% en poids de Pd et 210 mg d'acide orthophosphorique à 85%. En présence de 10 ppm d'hydroquinone après 3 h d'agitation à 150° C sous une pression d'hydrogène maintenue constante de 100 bar, le taux de conversion du propyl-2 dioxolanne atteint 99,9% et 74,7 g de butoxy-2 éthanol-1 (TT 89,9%) sont dosés par chromatographie. Le taux de transformation du propyldioxolanne en dibutoxy-1-2 éthane atteint 8,5% (5,2 g) et 2% en butanol normal (1,04 g).

*Exemple 21*

On opère dans les conditions de l'exemple 17. Le mélange soumis à l'hydrogénolyse contient 128,4 g (1,106 mol) de propyldioxolanne et 171,6 g d'éthylèneglycol, ce qui correspond à un rapport molaire $CH_2OH-CH_2OH$ sur

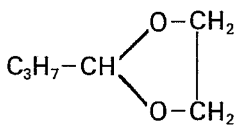 de 2,5.

Après 3 h d'agitation à 150° C sous une pression maintenue constante de 100 bar d'hydrogène, en présence du couple catalytique 1 g de $Pd/Al_2O_3$ à 5% de palladium et 210 mg d'acide phosphorique à 85%, la quantité de propyl-2 dioxolanne 1-3 transformée est de 117,5 g. Le poids de butoxy-2 éthanol-1 formé atteint 108,2 g (TT 83,5%) et celui en dibutoxy-1-2 éthane, impureté principale, de 8 g (0,046 mol).

*Exemple 22*

On opère selon l'exemple 1 en maintenant identiques toutes les proportions des réactifs et catalyseurs, mais en l'absence d'hydroquinone. Après 3 h de réaction à 150° C sous 100 bar d'hydrogène, l'autoclave est vidé et le mélange réactionnel filtré sous atmosphère d'azote. L'analyse chromatographique du filtrat montre que le taux de conversion totale du propyl-2 dioxolanne 1-3 atteint 99%. Le poids de butoxy-2 éthanol-1 formé est de 47,6 g (0,403 mol) et celui en dibutoxyéthane de 1,34 g. Les taux de transformation molaire du propyl-1 dioxolanne 1-3 en autres impuretés: dibutyléther, butyrate d'éthyle et butanol normal atteignent respectivement 0,17-1,1 et 1,75% contre 0,1-0,2 et 1,05% dans l'exemple 1 dans lequel on a opéré en présence de 10 ppm d'hydroquinone par rapport au mélange des réactifs PDX – éthylèneglycol.

*Exemple 23*

L'hydrogénolyse dans un réacteur agité de 1 l de capacité, à la température de 200° C sous une pression constante de 100 bar d'hydrogène, pendant 7 h, d'un mélange constitué de 50 g de propyl-2 dioxolanne et 250 g d'éthylèneglycol donne 46,3 g (0,392 mol) de butoxy-2 éthanol-1 en présence du couple: 1 g de catalyseur au palladium déposé sur alumine à raison de 5%, et 90 mg d'acide phosphorique à 85%. Dans ces conditions le taux de conversion du PDX atteint 97,2%.

*Exemple 24*

Le mélange constitué de 50 g (0,431 mol) de propyl-2 dioxolanne 1-3 et 250 g d'éthylèneglycol est hydrogénolysé dans un autoclave de 1 l agité mécaniquement en présence de 9 mg d'hydroquinone, 5 g de catalyseur au palladium déposé sur charbon à raison de 5% en poids de palladium et de 210 mg d'acide phosphorique concentré. Après 3 h à la température de 170° C sous une pression d'hydrogène maintenue constante de 30 bar, le taux de conversion du propyl-2 dioxolanne atteint 98,2%, 46,1 g (0,390 mol) de butoxy-2 éthanol-1 sont produits à côté de 2,35 g (0,0135 mol) de dibutoxy 1-2 éthane et de 0,31 g (0,004 mol) de butanol normal.

*Exemple 25*

Dans un autoclave en acier inoxydable de 1 l, 67,6 g de (cyclohexane-3) yl – 2 dioxolanne 1-3

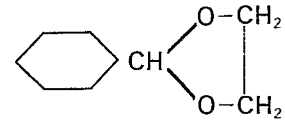

et 232,4 g d'éthylèneglycol sont introduits avec 9 mg d'hydroquinone et un couple catalytique constitué de 2 g de palladium déposé sur charbon à raison de 2% en poids de Pd et 210 mg d'acide orthophosphorique concentré à 85%. Après purge à l'azote, le réacteur agité est chauffé sous hydrogène à 170° C. La pression est ajustée à 100 bar et maintenue constante pendant 3 h. Après refroidissement et filtration pour éliminer le catalyseur hétérogène palladium sur charbon, le mélange réactionnel est analysé par chromatographie en phase gazeuse. 99,8% de l'acétal ont disparu et 35,6 g d'hydroxy-oxa-3 butyl cyclohexane

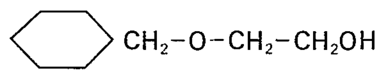

sont dosés par CPG.

*Exemple 26*

On opère selon les conditions de l'exemple 24. L'hydrogénolyse d'un mélange de 65 g d'(éthyl-1')pentyl-2 dioxolanne 1-3 et 235 g d'éthylèneglycol conduit à un taux de transformation de l'acétal en (éthyl-2')hexyloxy-2 éthanol-1

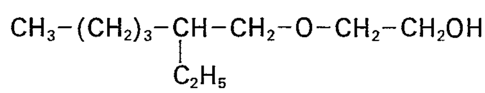

de 63,8% et un taux de conversion de l'acétal de 78,8%.

*Exemple 27*

On opère selon l'exemple 24. L'hydrogénolyse d'un mélange de 42,8 g (0,486 mol) de méthyldioxolanne-1,3 et 257,2 g d'éthylèneglycol conduit à la formation de 40,5 g (0,45 mol) d'éthoxy-2 éthanol-1, le taux de conversion de l'acétal étant de 99,8%.

*Exemple 28*

Les réactifs butyraldéhyde, 31,2 g (0,433 mol) et 268,8 g d'éthylèneglycol dans un rapport molaire 10 pour 1, l'hydroquinone: 9 mg, $H_3PO_4$ 85% et le palladium à 5% sur alumine à raison respectivement de 600 ppm et 0,33% en poids du mélange, sont chargés dans un réacteur en acier inoxydable,

puis chauffés sous agitation pendant 3 h à 100° C, l'autoclave ayant été préalablement purgé à l'azote. Sans ouvrir l'autoclave, l'hydrogène est introduit sous une pression de 100 bar et l'hydrogénolyse conduite à 185° C pendant 2 h en maintenant la pression d'hydrogène constante. Après refroidissement et filtration le mélange réactionnel est dosé par CPG. Dans les conditions expérimentales, le taux de conversion du butyraldéhyde atteint 99,2% et celui de l'éthylèneglycol 8,85% (99,4% de la quantité théorique à consommer). L'analyse montre que les produits principaux formés sont le butoxy-2 éthanol-1 avec un rendement molaire par rapport au butyraldéhyde consommé de 59,2% et le propyl-2 dioxolanne-1,3 avec un rendement par rapport au butyraldéhyde consommé de 23,8%. A côté des 30 g de butoxy-2 éthanol-1 et 11,85 g de

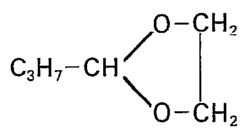

2,5 g (0,0338 mol) de butanol normal se forment. La somme des autres impuretés butyrate d'éthyle, dibutoxy-1,2 éthane et dibutyléther est inférieure à 0,7 g, soit 0,23% du poids du mélange des réactifs.

## Revendications

1. Procédé de fabrication de monoéther de monoéthylèneglycol par hydrogénolyse d'un acétal en présence d'un catalyseur, caractérisé en ce qu'elle est effectuée sur un dioxolanne 1-3 de formule

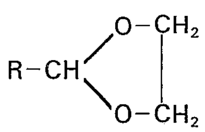

dans laquelle R est l'hydrogène ou un groupement hydrocarboné saturé aliphatique, linéaire ou ramifié, cycloaliphatique ou aromatique, substitué ou non par d'autres groupements hydrocarbonés, le nombre de carbone de la chaîne principale étant compris entre 1 et 12 et celui de chacun des substituants entre 1 et 4, avec comme système co-catalytique le palladium et un acide du phosphore ou un ester phosphorique de l'éthylèneglycol et en présence d'éthylèneglycol.

2. Procédé selon la revendication 1, caractérisé en ce que l'hydrogénolyse s'effectue à une température comprise entre 100 et 250° C.

3. Procédé selon l'une des revendications 1 ou 2, caractérisé en ce que l'hydrogénolyse s'effectue sous une pression de 20 à 150 bar.

4. Procédé selon l'une des revendications 1 à 3, caractérisé en ce que la quantité de palladium varie de 50 à 1500 ppm par rapport à la masse du mélange dioxolanne 1-3-éthylèneglycol.

5. Procédé selon l'une des revendications 1 à 4, caractérisé en ce que la teneur en acide du phosphore ou en ester phosphorique de l'éthylèneglycol rapporté en $H_3PO_4$ varie entre 0,01 et 0,75% de la masse du mélange dioxolanne 1-3-éthylèneglycol.

6. Procédé selon l'une des revendications 1 à 5, caractérisé en ce que le rapport molaire éthylèneglycol sur dioxolanne 1-3 varie entre 1 et 15.

7. Procédé selon l'une des revendications 1 à 6, caractérisé en ce que la réaction s'effectue en présence d'un stabilisant de la famille de l'hydroquinone.

## Patentansprüche

1. Verfahren zur Herstellung von Monoethylenglykolmonoether durch Hydrogenolyse eines Acetals in Gegenwart eines Katalysators, dadurch gekennzeichnet, dass sie mit einem 1,3-Dioxolan der Formel

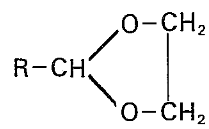

in der R Wasserstoff oder eine lineare oder verzweigte gesättigte aliphatische, cycloaliphatische oder aromatische Kohlenwasserstoffgruppe ist, die durch andere Kohlenwasserstoffgruppen substituiert oder unsubstituiert ist, wobei die Anzahl der Kohlenstoffatome der Hauptkette zwischen 1 und 12 liegt und die der einzelnen Substituenten zwischen 1 und 4, mit Palladium und einer Phosphorsäure oder einem Phosphorsäureester des Ethylenglykols als cokatalytischem System und in Gegenwart von Ethylenglykol durchgeführt wird.

2. Verfahren nach Anspruch 1, dadurch gekennzeichnet, dass die Hydrogenolyse bei einer Temperatur zwischen 100 und 250° C durchgeführt wird.

3. Verfahren nach einem der Ansprüche 1 oder 2, dadurch gekennzeichnet, dass die Hydrogenolyse bei einem Druck zwischen 20 und 150 bar durchgeführt wird.

4. Verfahren nach einem der Ansprüche 1 bis 3, dadurch gekennzeichnet, dass die Palladiummenge zwischen 50 und 1500 ppm, bezogen auf die Masse des Gemischs 1,3-Dioxolan/Ethylenglykol, beträgt.

5. Verfahren nach einem der Ansprüche 1 bis 4, dadurch gekennzeichnet, dass der Gehalt an Phosphorsäure oder Phosphorsäureester des Ethylenglykols, angegeben als $H_3PO_4$, zwischen 0,01 und 0,75% der Masse des Gemischs 1,3-Dioxolan/Ethylenglykol beträgt.

6. Verfahren nach einem der Ansprüche 1 bis 5, dadurch gekennzeichnet, dass das Molverhältnis zwischen Ethylenglykol und 1,3-Dioxolan zwischen 1 und 15 beträgt.

7. Verfahren nach einem der Ansprüche 1 bis 6, dadurch gekennzeichnet, dass die Reaktion in Gegenwart eines Stabilisierungsmittels aus der Familie des Hydrochinons durchgeführt wird.

## Claims

1. Process for the manufacture of monoethylene glycol monoether by hydrogenolysis of an acetal in the presence of a catalyst, characterized in that it is carried out with a 1,3-dioxolane of formula

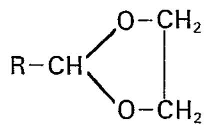

in which R is hydrogen or a saturated aliphatic, straightchain or branched, alicyclic or aromatic hydrocarbon group, which is unsubstituted or substituted by other hydrocarbon groups, the carbon number of the main chain being between 1 and 12 and that of each of the substituents between 1 and 4, with, as a co-catalyst system, palladium and an acid of phosphorus or a phosphoric ester of ethylene glycol and, in the presence of ethylene glycol.

2. Process according to Claim 1, characterized in that the hydrogenolysis is carried out at a temperature between 100 and 250° C.

3. Process according to one of Claims 1 to 2, characterized in that the hydrogenolysis is carried out under a pressure of 20 to 150 bar.

4. Process according to one of Claims 1 to 3, characterized in that the quantity of palladium varies from 50 to 1500 ppm relative to the mass of the 1,3-dioxolane/ethylene glycol mixture.

5. Process according to one of Claims 1 to 4, characterized in that the content of the acid of phosphorus or of a phosphoric ester of ethylene glycol, expressed as $H_3PO_4$, varies between 0.01 and 0.75% of the mass of the 1,3-dioxolane/ethylene glycol mixture.

6. Process according to one of Claims 1 to 5, characterized in that the molar ratio ethylene glycol over 1,3-dioxolane varies between 1 and 15.

7. Process according to one of Claims 1 to 6, characterized in that the reaction is carried out in the presence of a stabiliser of the hydroquinone group.